# EUROPEAN PATENT SPECIFICATION

(11) **EP 4 337 297 B1**
(45) Date of publication and mention of the grant of the patent: **22.07.2026**
(21) Application number: 22728539.2
(22) Date of filing: 10.05.2022
(51) Int. Cl.: A61M 37/00

(54) **MICRONEEDLE BASED DELIVERY SYSTEM**
MIKRONADELBASIERTES VERABREICHUNGSSYSTEM
SYSTÈME D'ADMINISTRATION BASÉ SUR DES MICRO-AIGUILLES

(30) Priority: 10.05.2021 EP 21173092
(43) Date of publication of application: 20.03.2024
(73) Proprietor: Latch Medical Limited, 4 Dublin (IE)
(72) Inventor: BERTOLLO, Nicky, Dublin (IE)
(74) Representative: FRKelly
(86) International application number: PCT/EP2022/062653
(87) International publication number: WO 2022/238411

(56) References cited:
- WO-A1-2012/006677
- US-A1- 2019 134 370

## Description

### Field of the invention

This invention relates to a microneedle based delivery system, in particular such a system for drug or vaccine delivery to target tissue such as skin tissue with accuracy and ease of use.

### Background of the invention

Microneedles are gaining increased use in various medical applications in view of the many documented benefits for both patients and medical professionals, for example reduced tissue trauma, surgery times and patient recovery, reduced risk of infection, and minimising the surgical or medical equipment needed in procedures involving microneedle based devices. Such microneedles allow for extremely accurate, and shallow, deployment into tissue, which can be beneficial in various applications, for example in drug delivery, and more particularly in drug delivery to sensitive or delicate tissue such as skin, ocular tissue and oral mucosa.

International patent applications WO2018/069543 and WO2019/201903 provide detailed disclosures of the configuration and operation of microneedles and opposing microneedle arrays which may be provided in the form of a device for application to a tissue substrate for various surgical and therapeutic uses, one particular use being drug delivery directly from or through the microneedles provided.

WO2012006677 discloses an apparatus for applying a patch to a subject, the patch having a number of projections thereon, the apparatus including opposable jaws movable between open and engaging positions, wherein in the open position the jaws can receive at least part of the subject, and in the engaging position the jaws can engage the at least part of the subject. US2019134370 discloses a microneedle puncture instrument including a pinch section including a first pinch element and a second pinch element and a puncture section configured to puncture the skin while pinching the skin, the puncture section including a microneedle for puncturing the skin.

Intradermal delivery of drugs and vaccines offers significant advantages over conventional intramuscular and oral delivery routes for vaccines and drugs. Systemic uptake of therapeutics via the dermal blood capillaries and lymphatic system offers a host of benefits, including avoiding the deleterious effects of first-pass metabolism, rapid drug onset and improved bioavailability of APIs, such as biologics, that are not readily absorbed across the mucosal layers of the gastrointestinal tract. Additionally, the dermal layer of skin is replete with antigen presenting cells and therefore represents an optimal location for the delivery of vaccines in order to elicit an enhanced immune response, and in some cases, using lower dosages relative to standard intramuscular administration.

The viscoelastic and highly deformable nature of skin poses significant clinical challenges in the delivery of liquid formulations of both low and high volume and low and high viscosity (such as can be the case for certain of biologics) to specific depths in skin using hollow microneedle and hypodermic needle-based approaches. Uncontrolled deformation of the skin significantly limits accuracy of depth targeting, which is further compounded by natural inter- and intra-subject anatomical variability in thickness and biomechanical properties of skin. Skin deformation and compression caused by placement and application of these devices increases the injection pressure requirements, impacts flow-rate, significantly limits the physical volumes and viscosities that can be delivered and also impairs intradermal bleb/bolus diffusion kinetics. Furthermore, the absence of intrinsic anchorage of microneedle technologies, in particular, during intradermal injection requires additional mechanical work be manually applied to the injection device or patch to counter the injection pressure. Unless balanced, this component of injection pressure is driving to eject and cause relative migration of skin and/or needle tips, affecting the site of delivery but which can also result in formulation leakage, efflux and spray back. Appreciably, operator movement(s) during handling and injection is another factor further impacting depth targeting accuracy and dose delivery, where the absence of intrinsic anchoring can facilitate gross movements of the needle tips relative to the skin and distribute the injection away from the intended target depth in skin.

The layered structure of the eye, in addition to the delicate nature of the ocular tissue and the high potential for damage or complications during any form of ocular surgery or therapy, has presented difficulties in the targeted delivery of drugs to the eye, in particular where it is required to deliver the drug to a particular layer within the eye in order to improve efficacy and potentially avoid side effects which may arise when a drug is delivered to an unintended part of the eye. Conventional drug delivery to the eye is complicated through natural and, significant variability in the thickness of the sclera (outermost layer) of the eye. In addition, the eye readily rotates during injection, requiring stabilisation, and there is deformation of the eye tissue during injection which limits the ability to target specific regions of the eye. Relative movement of the injector and drug efflux during injection also occur, further complicating the procedure and compromising the efficiency of payload delivery.

It is therefore an object of the present invention to provide a self-anchoring microneedle based delivery system which is operable to quickly and easily deploy microneedles into a tissue substrate such as the skin and eye, for the purposes of targeted drug delivery of both low and high volumes of low and high viscosity solutions (such as biologics).

### Summary of the invention

According to the present invention there is provided a microneedle based delivery system comprising a body having a first section and a second section displaceable relative to one another; at least one first hollow microneedle provided on the first section and at least one second hollow microneedle provided on the second section, the first and second sections being displaceable relative to one another in order to transition the microneedles between a disengaged state and an engaged state; and a delivery manifold in fluid communication with the first and second hollow microneedles; wherein the first and second section of the body each define an elongate arm at a free end of which the respective at least one microneedle is provided and each arm defines an upper end opposite the lower end, and a fluid flow path extending through the arm between the upper and lower end, the delivery manifold being in fluid communication with the fluid flow path at the upper end of each arm.

Preferably, a longitudinal axis of the at least one first microneedle extends at a first oblique angle relative to the direction of displacement between the first and second sections, and a longitudinal axis of the at least one second microneedle extends at a second oblique angle relative to the direction of displacement between the first and second sections.

Preferably, the first oblique angle extends away from the second oblique angle.

Preferably, the at least one first microneedle is transversely offset to the at least one second microneedle relative to the direction of displacement between the first and second sections.

Preferably, the first and the second sections are slidably and/or hingedly displaceable relative to one another.

Preferably, the delivery manifold is captured between the first and second sections at least when the microneedles are in the engaged state.

Preferably, the delivery manifold is clamped against the body when the microneedles are in the engaged state such as to establish a fluid tight seal between the delivery manifold and the body.

Preferably, the delivery manifold comprises an inlet adapted for connection with a fluid reservoir and an outlet engagable with the body such that the inlet is in fluid communication with the hollow microneedles.

Preferably, the body defines a chamber between the first and second sections in which the outlet of the delivery manifold is captured and which chamber is arranged to bias the outlet into sealing engagement with the body at least when the system is in the engaged state.

Preferably, the body defines an enclosure at least partially surrounding the delivery manifold.

Preferably, the delivery manifold defines a first fluid flow path in fluid communication with the at least one first microneedle and an independent second fluid flow path in fluid communication with the at least one second microneedle.

Preferably, the delivery manifold is in fluid communication with the end of the fluid flow paths when the microneedles are in both the engaged and disengaged state.

Preferably, the microneedle based delivery system comprises a release mechanism defined by a trigger on each of the first and second sections and arranged to facilitate manual displacement of the system into the disengaged state.

Preferably, the microneedle based delivery system comprises a locking mechanism to releasably secure the system in the engaged state.

Preferably, the locking mechanism is integrated with at least one of the triggers and is releasable through actuation of the trigger.

Preferably, the at least one trigger with which the locking mechanism is integrated is resiliently deformable.

Preferably, the microneedle based delivery system comprises a stop releasably engageable with the body to limit relative displacement between the first and second sections.

Preferably, the delivery system comprises a retention lock operable to prevent the first and second sections from being separated from one another beyond the disengaged state.

Preferably, the delivery system comprises a pair of tissue contacting feet each defining a tissue contacting surface which is substantially longitudinally aligned with the first and second hollow microneedles.

Preferably, the microneedle based delivery system comprises interlocking elements operable to prevent the first and second sections from being displaced from the disengaged to the engaged state when the elements are interlocked, and which are separable in response to downward pressure and reactive forces applied to the feet by contacted tissue.

### Brief description of the drawings

The present invention will now be described with reference to the accompanying drawings, in which:
Figure 1 illustrates a perspective view of a microneedle based delivery system according to an embodiment of the present invention, coupled with a syringe, and in a disengaged state;
Figure 2 illustrates a front elevation of the delivery system as shown in Figure 1:
Figure 3 illustrates a front elevation of the delivery system in an engaged state;
Figure 4 illustrates a cut away perspective view of the delivery system in the disengaged state;
Figure 5 illustrates a cut away perspective view of the delivery system in the engaged state;
Figure 6 illustrates an exploded perspective view of the delivery system from a first side;
Figure 7 illustrates the exploded perspective view of Figure 6 from the reverse side;
Figure 8a illustrates a sectioned front elevation revealing a release mechanism when the system is in the engaged state;
Figure 8b illustrates the arrangement of Figure 8a with the release mechanism being activated;
Figure 8c illustrates the arrangement of Figure 8a when the system is in the disengaged state;
Figure 9 illustrates a perspective view of a microneedle based delivery system according to an alternative embodiment of the present invention and in a disengaged state;
Figure 10 illustrates a front elevation of the delivery system as shown in Figure 9;
Figure 11 illustrates a front elevation of the delivery system of Figures 9 and 10 in an engaged state;
Figure 12 illustrates a cut away view of the delivery system of Figures 9 to 11 in the engaged state;
Figure 13 illustrates a front elevation of a microneedle based delivery system according to a further alternative embodiment of the present invention and in a disengaged state;
Figure 14 illustrates a front elevation of the delivery system as shown in Figure 13 in a partially engaged state;
Figure 15 illustrates a front elevation of the delivery system of Figures 13 and 14 in a fully engaged state;
Figure 16 illustrates a perspective view of a stop for location within the delivery system of Figures 13 to 15 to facilitate selective limitation of displacement between first and second sections of the system;
Figure 17 illustrates a perspective view of a microneedle based delivery system according to a further embodiment of the present invention in a disengaged state;
Figure 18 illustrates a side elevation of the delivery system as shown in Figure 17:
Figure 19 illustrates a perspective view of the microneedle based delivery of Figure 17 in an engaged state;
Figure 20 illustrates a side elevation of the delivery system as shown in Figure 19;
Figure 21 illustrates an exploded perspective view of the delivery system of Figures 17 to 20;
Figure 22 illustrates a side elevation of the delivery system of Figures 17 to 21 with a pair of halves substantially separated from one another;
Figure 23 illustrates an end elevation of the delivery system of Figures 17 to 22;
Figure 24 illustrates a perspective view of one half or section of the delivery system of Figures 17 to 23 as seen from one side thereof;
Figure 25 illustrates an alternative perspective view of the section shown in Figure 24;
Figure 26 illustrates a perspective view of an other section of the delivery system for interlocking engagement with the section of Figure24;
Figure 27 illustrates the halves or sections shown in Figure 24-26 in a designed for manufacturing form factor;
Figure 28 illustrates the section of Figure 27 separated into two constituent parts;
Figure 29 illustrates a perspective view of a microneedle based delivery system according to another embodiment of the present invention in a partially disassembled or separated state;
Figure 30 illustrates the delivery system as shown in Figure 29 advanced into a disengaged state:
Figure 31 illustrates an end elevation of the microneedle based delivery of Figures 29 and 30; and
Figure 32 illustrates a plan view of the delivery system of Figures 29 to 31 in an engaged state.

### Detailed description of the drawings

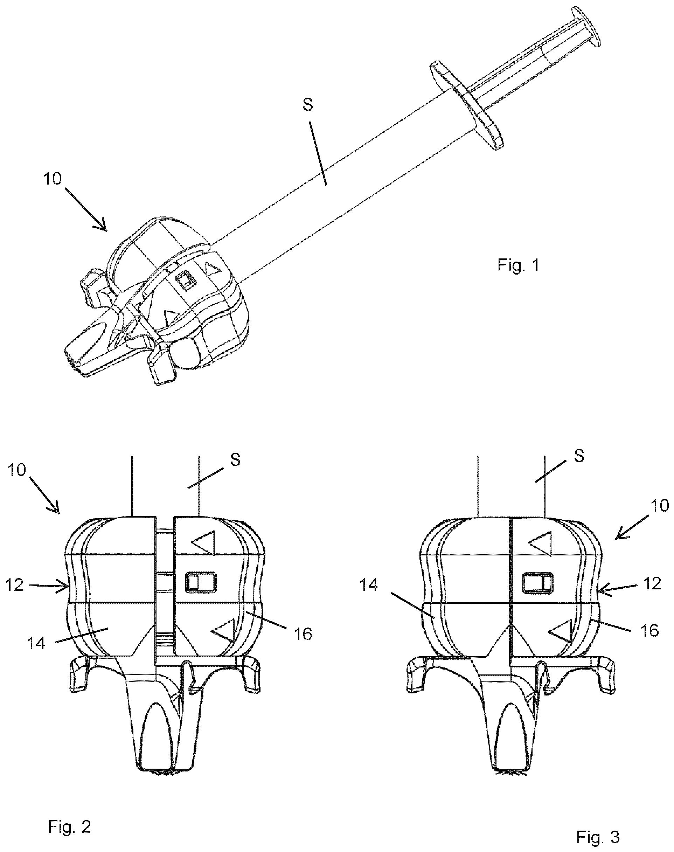

Referring now to Figures 1 to 8 of the accompanying drawings there is illustrated a microneedle based delivery system, generally indicated as 10, for use in delivering one or more fluids, in particular a dose of a drug or therapeutic agent in liquid form, to a target area of tissue. The delivery system 10 is suitable for use with a wide range of tissue, for example skin or muscle, but is particularly suited for use in delivering a drug to ocular tissue such as the subchoroidal or suprachoroidal regions of the eye (not shown). The delivery system 10 is also preferably adapted to be coupled to an external fluid supply, most preferably a conventional syringe S as will be described hereinafter, to allow a fluid to be dispensed from the syringe S or other external fluid supply to the target tissue.

The delivery system 10 comprises a body 12 having a first section 14 and a second section 16 which are respectively provided with at least one first hollow microneedle 18 and at least one second hollow microneedle 20 which are adapted to be reversibly insertable into the target tissue to allow for drug delivery through the hollow microneedle 18, 20, the operation of which will be described in detail hereinafter. The number of microneedles 18, 20 may be varied, for example to suit a particular application, target area, drug to be delivered and/or delivery rate, and it is also envisaged that one or more solid microneedles (not shown) may be provided to securely anchor the hollow microneedles 18, 20 to the target tissue to achieve reliable drug delivery. The material, dimensions, orientation, and relative positioning of the microneedles 18, 20 may also be varied as necessary. For example the dimension and/or orientation of the microneedles 18, 20 may be arranged to provide a desired depth of insertion into the target tissue, which can therefore facilitate accurate drug delivery to specific locations or layers of tissue.

In a preferred embodiment the body 12 is substantially formed from one or more polymers and the delivery system 10 is intended as a single use product. The microneedles 18, 20 may also be formed from a polymer, or may be metal or another material and suitably secured to the first and second section 14, 16. However it is also envisaged that the system 10 could be substantially formed from a metal such as stainless steel, titanium, etc. or a hard wearing polymer and may be reusable following sterilisation, for example in an autoclave. The microneedles 18, 20 could be provided as a modular component releasably securable to the body 12 and thus single use while the body 12 is reusable.

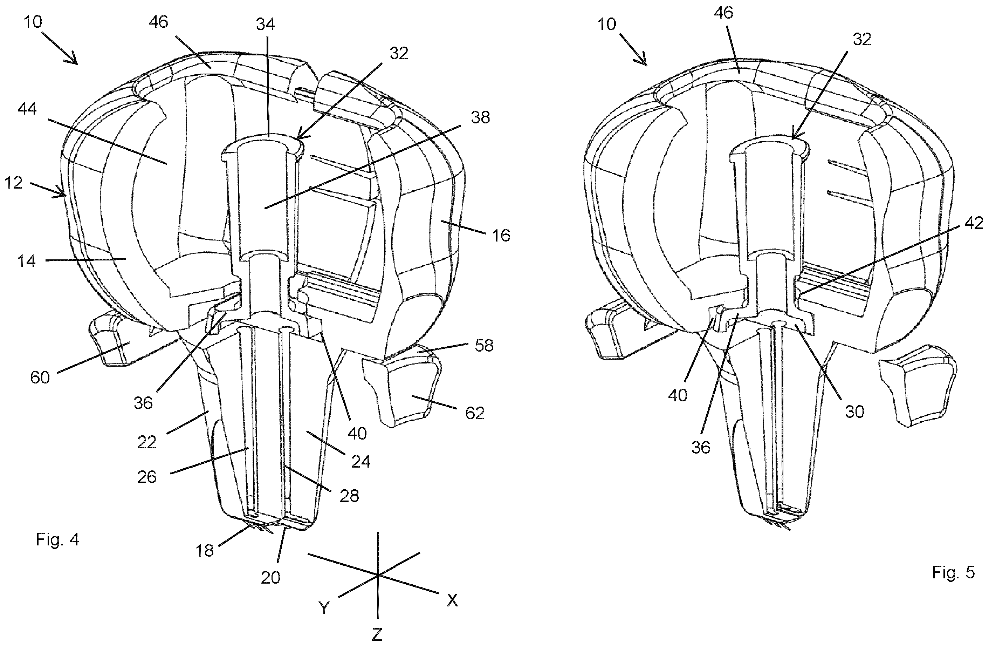

The first and second sections 14, 16 of the body 12 are secured together but displaceable relative to one another by a fixed distance, and in the embodiment illustrated are slidably displaceable relative to one another to transition the system 10, and in particular the microneedles 18, 20 between a disengaged state, for example as illustrated in Figures 1, 2 and 4, and an engaged state, for example as illustrated in Figures 3 and 5. In the disengaged stage the microneedles 18, 20 are in a first orientation relative to one another prior to application to the target tissue, and in the engaged state are in a second orientation relative to one another. In use the microneedles 18, 20 are applied against the target tissue in the disengaged state and the system 10 is then displaced into the engaged state by manually advancing the first and second section 14, 16 towards one another, which action effects the relative displacement of the microneedles 18, 20 in a manner which draws and anchors the microneedles 18, 20 into the target tissue to allow drug delivery as hereinafter described.

The underlying methodology of this deployment technique is described in detail in the above mentioned international applications WO2018/069543 and WO2019/201903. For ease of reference, hereinafter directions or dimensions in a direction between the first and second sections 14, 16 of the delivery system 10 and the microneedles 18, 20 will be referred to as being an "X" coordinate or direction, transversely or along the width will be referred to as being a 'Y" coordinate and along the depth will be referred to as being a "Z" coordinate, and as represented schematically in Figure 4 relative to the delivery system 10.

In order to allow the microneedles 18, 20 to be accurately positioned, particularly in the case of tissue that is relatively difficult to accurately engage or pierce, the body 12 comprises a first arm 22 extending from the first section 14 and an adjacent second arm 24 extending from the second section 16, both of which are elongate in form in the "Z" or depth direction, which is beneficial for engaging with relatively inaccessible tissue such as ocular tissue. The at least one first microneedle 18 is provided on a lower end of the first arm 22 while the at least one second microneedle 20 is provided on a lower end of the second arm 24. In the embodiment illustrated the arms 22, 24 taper inwardly as they extend away from the body 12 towards the microneedles 18, 20, thereby defining a relatively small footprint at the lower end on which the microneedles 18, 20 are located, allowing both accurate location and good visibility of the microneedles 18, 20 and surrounding tissue during application to the target tissue. It will of course be appreciated that the shape, orientation and dimensions of the arms 22, 24 may be varied as required, in particular depending on the application of the delivery system 10 and/or the type and/or location of the target tissue..

In the embodiment illustrated the first and second arms 22, 24 are located adjacent one another, but offset in the "Y" direction, transverse to the "X" direction in which the first and second sections 14, 16 are displaceable relative to one another. In this way the arms 22, 24 can move into overlapping alignment as the system 10 is transitioned into the engaged state, drawing the microneedles 18, 20 into the tissue, as can be seen in particular in Figures 4 and 5. However other geometries and arrangements are also possible, for example one arm could be provided with a channel to at least partially receive the other arm when in the engaged state, or the arms may remain separated or spaced from one another in both the disengaged and engaged states.

In order to allow drug delivery through the hollow microneedles 18, 20 at least the first arm 22 is provided with a first delivery conduit 26 extending internally along the length of the arm 22 (in the "Z" direction) and in fluid communication with the at least one first microneedle 18. In the preferred embodiment illustrated the second arm 24 is provided with a second delivery conduit 28 extending internally along the length of the second arm 24 and in fluid communication with the at least one second microneedle 20. It is to be understood that the system 10 could function with a single delivery conduit to supply only the first or second microneedles 18, 20, but it is preferred that both the hollow microneedles 18, 20 can be supplied with and used for drug delivery to the target tissue.

The first and second arms 22, 24 each terminate at an upper end or face 30 onto which the delivery conduits 26, 28 open, thereby establishing a fluid flow path from the upper face 30 through the arms 22, 24 to the microneedles 18, 20. Referring to Figure 4 it can be seen that when the system 10 is in the disengaged state the upper ends of the delivery conduits 26, 28 are spaced from one another in the "X" direction, and when the system 10 is transitioned into the engaged stage as illustrated in Figure 5 the upper ends of the delivery conduits 26, 28 are adjacent one another and thus overlapping or aligned in the "X" direction. Located above and enclosing the upper ends of the deliver conduits 26, 28 is a delivery manifold 32 which comprises an inlet 34, an outlet 36 and a lumen 38 extending therebetween. The outlet 36 is in register with the upper faces 30 such as to enclose the upper ends of the delivery conduits 26, 28, thereby allowing fluid delivery through the delivery manifold 32, into the delivery conduits 26, 28, and ultimately to the microneedles 18, 20. The inlet 34 is adapted to be releasably secured to a convention luer lock connector or head H of the syringe S, but may be adapted for a fluid tight connection to any other desired external fluid supply (not shown). It will therefore be understood that the syringe S, containing a drug or other fluid to be delivered, can be connected to the system 10 via the delivery manifold 32, and fluid can thus be dispensed from the syringe S, through the microneedles 18, 20 to the target tissue in a reliable and accurate manner.

In order to secure the delivery manifold 32 to the body 12 the delivery manifold 32, and most preferably the outlet 36, is captured between the first and second sections 14, 16 at least when the microneedles 18, 20 are in the engaged state. In the embodiment of Figures 1 to 8 the body 12 defines a chamber 40 located directly above and partially defined by the upper faces 30, one part or side of the chamber 40 formed in the first section 14 and the other side of the chamber 40 being formed in the second section 16 opposite one another. The chamber 40 is shaped and dimensioned to encapsulate the outlet 36, with an opening 42 being provided in an upper wall of the chamber 40 through which the delivery manifold 32 extends into an enclosure 44 defined by the body 12. The enclosure 44 is preferably shaped and dimensioned to surround and effectively enclose the delivery manifold 32 such that, in use, the delivery manifold 32, and therefore the connection to the syringe S, is inaccessible. A mouth 46 is formed in an upper region of the enclosure 44 to accommodate the syringe S. As the mouth 46 extends across the interface between the first and second sections 14, 16 it will be appreciated that with the system 10 in the disengaged state the mouth 46 will be enlarged to allow the syringe S to be advanced into the chamber 44 to couple the head H with the inlet 34 of the delivery manifold 32. When the system 10 is transitioned into the engaged state the mouth 46 will partially close about the body of the syringe S to avoid any unintentional movement or uncoupling of the syringe S.

At the interface between the outlet 36 of the delivery manifold 32 and the upper face 30 of the arms 22, 24, as seen in particular in Figures 4 and 5, the outlet 36 is dimensioned to enclose the upper ends of the delivery conduits 26, 28 when the system 10 is in both the disengaged state of Figure 4 and the engaged state of Figure 5, although the system 10 could be adapted such that the outlet 36 only encloses the upper ends of the delivery conduits 26, 28 when the system 10 is in the engaged state. However, it is often necessary to prime a syringe prior to dispensing a fluid, in order to ensure there is no air in the syringe that would then be injected into the subject. Thus by having the outlet 36 dimensioned to enclose the upper ends of the delivery conduits 26, 28 when the system 10 is in the disengaged state the syringe S can be primed or purged of air before application of the microneedles 18, 20 to the target tissue, as a fluid flow path exists between the syringe S and the microneedles 18, 20. When the microneedles 18, 20 are secured to the target tissue the system 10 will be in the engaged state, and as the outlet 36 also encloses the upper ends of the delivery conduits 26, 28 in this state the contents of the syringe S can then be delivered to the target tissue via the microneedles 18, 20.

It is envisaged that the manifold 32 could be replaced with a modified manifold (not shown) defining a pair of independent fluid flow paths each arranged in fluid communication with one of the delivery conduits 26, 28, thereby allowing two different fluids to be delivered to the first and second microneedles 18, 20 via a modified syringe (not shown) or pair of syringes (not shown) or other fluid supplies.

In order to prevent unwanted leakage of the contents of the syringe S at the interface between the outlet 36 and the upper face 30 of the arm 22, 24 a suitable fluid tight seal is established at the interface. This seal may be achieved in a number of ways, for example using gaskets, deformable rubber/elastomer seals, coatings, sealing geometries, mechanical interlocking or an interference fit between the outlet 36 and the upper face 30 of the arms 22, 24. In the embodiment of Figures 1 to 8 this seal is established by complimentary dimensioning of the outlet 36 and the chamber 40, in particular in the "Z" direction whereby the height or "Z" dimension of the chamber 40 is such that the upper wall of the chamber 40 contacts the upper wall of the outlet 36 and biases the outlet 36 against the upper face 30 of the arms 22, 24 such as to establish a seal therebetween. In a particularly preferred arrangement, the upper wall of the chamber 40 and the upper wall of the outlet 36, which are in face to face engagement, have a corresponding draft or incline, for example in the region of 1° or 2°, optionally up to 5° or 10° or more in the "X" direction of relative displacement between the first and second sections 14, 16. In this way, as the first and second sections 14, 16 are displaced towards one another in transitioning the system 10 into the engaged state, the bias applied to the outlet 36 will increase, thereby increasing the seal at the interface between the outlet 36 and the upper face 30 of the arms 22, 24. This arrangement increases the mechanical compressive force acting on the outlet 36 from nominal in the disengaged state to maximal in the engaged state. This allows for the above described priming of the syringe S and microneedles 18, 20 prior to injection into tissue, where pressures are low, to allowing higher pressures associated with the injection once the microneedles 18, 20 have been deployed into the tissue, without fear of leakage.

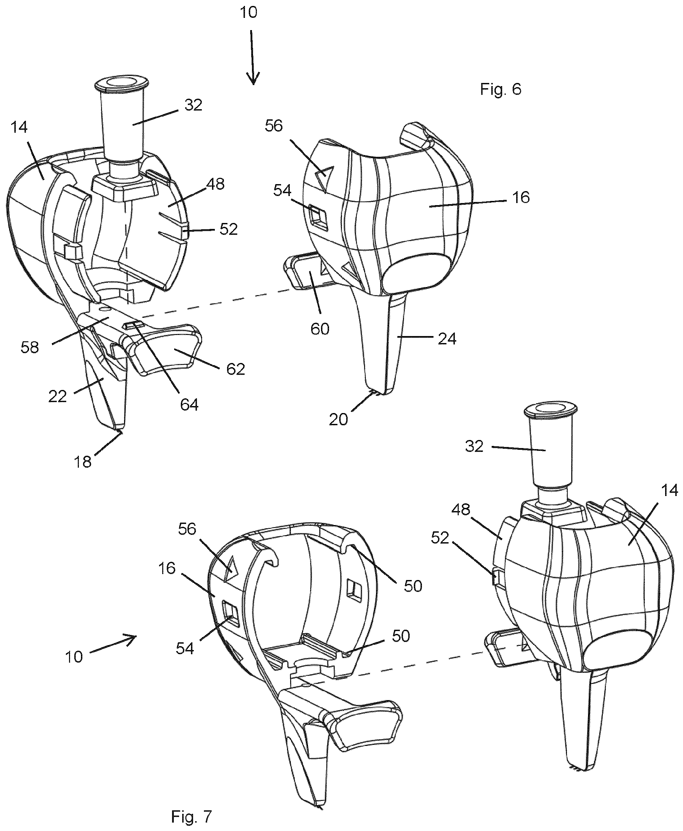

In use the delivery system 10 is transitioned from the disengaged state to the engaged state by manually applying pressure to the first and second sections 14, 16 of the body 12, preferably via the outer walls of the enclosure 44. As seen in Figures 6 and 7 the walls of body 12 which define the enclosure 44 include extensions 48 on the first section 14 and corresponding guideways 50 on the second section 16 which interlock to secure the sections 14, 16 together while permitting limited relative displacement. Tabs 52 are provided in the extensions 48 which will be visible through corresponding windows 54 when the system 10 has been correctly transitioned into the engaged state to provide a visual indication to the user signifying that the microneedles 18, 20 are embedded and fluid delivery from the syringe S can be undertaken. The tabs 52 further serve to ensure that the first and second sections 14, 16 can not disengage from one another. Graphical indicia 56 may be provided about the body 12 to indicate the correct direction in which the first and second sections 14, 16 should be displaced to transition the system 10 into the engaged state. The system 10 may be provided with a stop (not shown) releasably engageable with the body 12, for example located within the enclosure 44, to limit relative displacement between the first and section sections 14, 16 as a means of limiting the depth of penetration of the microneedles 18, 20 to suit a particular application or anatomical consideration.

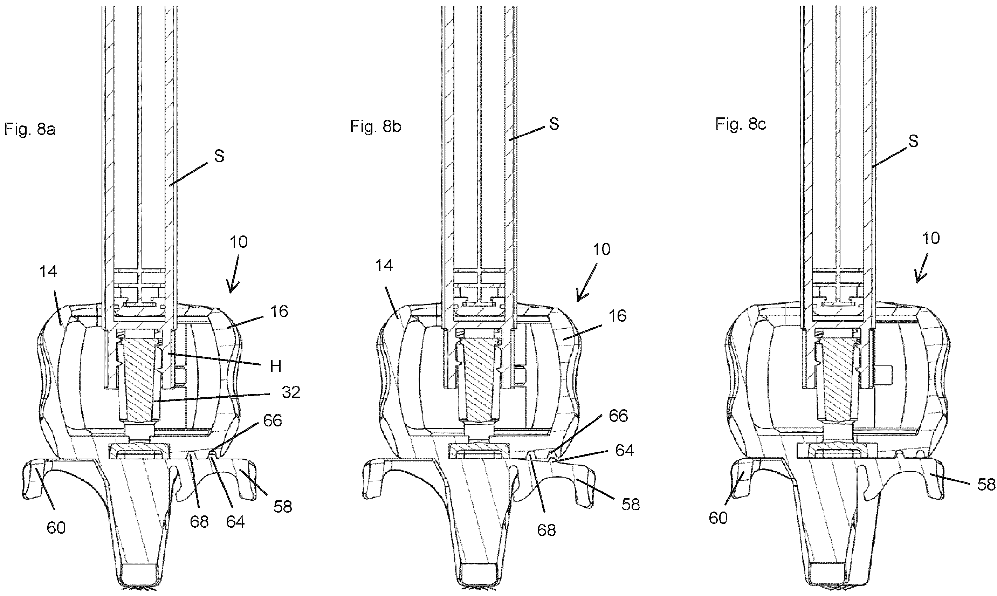

Once delivery of fluid to the target tissue has been completed it is necessary to disengage the system 10, in particular the microneedles 18, 20, from the tissue. It is therefore necessary for the user to displace the first and second sections 14, 16 away from one another to transition the system 10 into the disengaged state and thus affect withdrawal of the microneedles 18, 20. In order to facilitate this action the system 10 comprises a release mechanism in the form of first and second triggers 58, 60 respectively provided on the first and second sections 14, 16, in the region of the upper face 30 of the arms 22, 24 and positioned beneath the enclosure 44. Each trigger 58, 60 extends in cantilever form towards and marginally beyond the wall of the enclosure 44 of the opposite section 14, 16 in order to be accessible by the user. Each trigger 58, 60 terminates in an enlarged paddle 62 which is shaped and dimensioned for operative engagement with a finger or thumb of the user. The triggers 58, 60 are therefore positioned side by side and slide relative to one another when the system 10 is displaced between the disengaged and engaged states without hindering said displacement of the system 10. Referring to Figure 6 the system 10 is preferably provided with a locking mechanism to secure the system 10 in the engage and or disengaged state, in particular to avoid any inadvertent withdrawal of the microneedles 18, 20, for example while fluid is being delivered to the tissue. In the embodiment illustrated the locking mechanism is provided in the form of a protrusion or detent 64 on an upper face of the first trigger 58 and a correspondingly shaped and dimensioned first socket 66 and second socket 68 on the underside of the enclosure 44 which is in face to face with the upper face of the first trigger 58, and visible in Figures 8a, 8b and 8c. The first and second sockets 66, 68 are spaced apart from one another by the distance through which the first and second sections 14, 16 are displaceable. The first socket 66 is located to receive the detent 64 when the system 10 is in the engaged state as illustrated in Figure 8a, to releasably secure the system 10 in the engaged state.

Referring in particular to Figures 8a, 8b and 8c the sequence of steps to effect displacement of the system 10 from the engaged to disengaged state is illustrated. Figure 8a shows the system 10 in the engaged state with the detent 64 captured in the first socket 66. In order to begin displacing the first and second sections 14, 16 away from one another the system 10 is gripped by the triggers 58, 60, for example between thumb and forefinger. At least the first trigger 58 is resiliently deformable, and the user therefore applies pressure to the first trigger 58 via the paddle 62 to deform the first trigger 58 in a manner which will draw the detent 64 out of the first socket 66 as illustrated in Figure 8b. At this point the user can apply pressure to both triggers 58, 60 to effectively push the triggers 58, 60 towards one another. This will effect relative displacement between the first and second sections 14, 16 to transition the system 10 into the disengaged state as shown in Figure 8c, drawing the microneedles 18, 20 out of the tissue. In this configuration the detent 64 will be aligned with the second socket 68 and once pressure is released the first trigger 58 will return and the detent 64 will enter the second socket 68 to lock the system 10 in the disengaged state. The system 10 can then be withdrawn from the tissue.

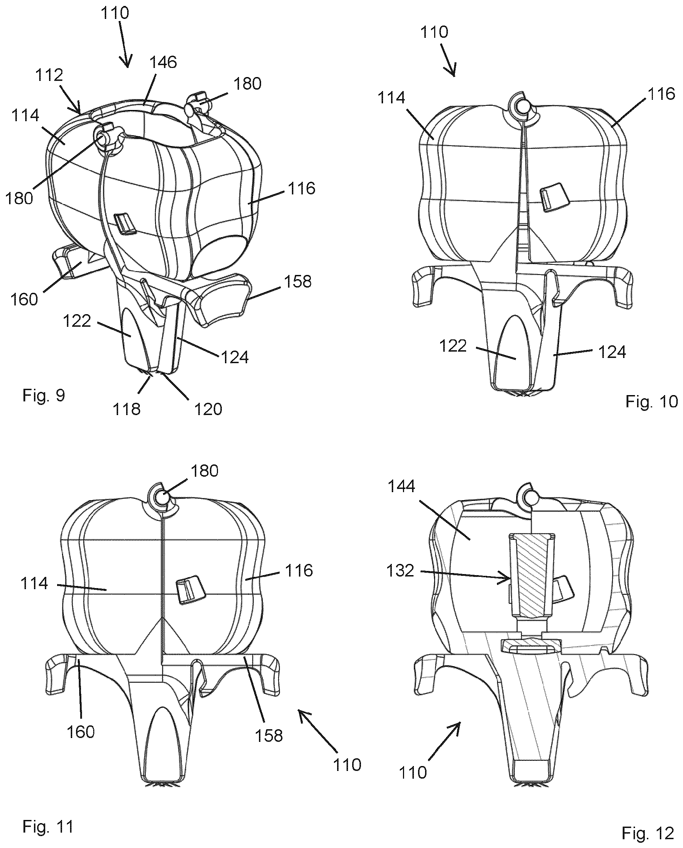

Referring now to Figures 9 to 12 there is illustrated an alternative embodiment of a microneedle based delivery system according to the present invention, generally indicated as 110. In this alternative embodiment like components have been accorded like reference numerals and unless otherwise stated perform a like function.

The delivery system 110 comprises a body 112 having a first section 114 and a second section 116 which are displaceable relative to one another to transition the system 110 between disengaged and engaged states. The system 110 comprises first hollow microneedles 118 provided on the first section 114 and second hollow microneedles 120 provided on the second section 116. The body 112 defines elongate first and second arms 122, 124 at a free end of which the microneedles 118, 120 are provided. Although not illustrated, first and second delivery conduits (not shown) extend through the arms 122, 124 into fluid communication with the microneedles 118, 120. A delivery manifold 132 is captured between the first and second sections 114, 116 to facilitate the coupling of a syringe (not shown) or other fluid supply to the system 110 for delivery through the microneedles 118, 120 in the same manner as described above in relation to the system 10 shown in Figures 1 to 8. The body 112 defines an enclosure144 above the arms 122, 124 in which the delivery manifold 132 is contained and which is accessible through a mouth 146 at an upper portion of the enclosure 144.

The overall composition, appearance and general operation of the system 110 is the same as described above with reference to the system 10 shown in Figures 1 to 8. However, unlike the system 10 the first and second sections 114, 116 of the system 110 are pivotally displaceable relative to one another in order to transition the system 110 between the disengaged and engaged states. The first and second sections 114, 116 are secured to one another by means of a pair of hinges 180 which are located on opposite sides of the mouth 146, although it will be appreciated that numerous other configurations may be employed to enable this pivoting relative displacement.

Thus as the first and second sections 114, 116 are displaced towards the engaged state the microneedles 118, 120 will move relative to one another along an arc, advancing the microneedles 118, 120 into the target tissue. Once the system 110 is in the engaged stage with the microneedles 118, 120 embedded in the tissue a fluid can be delivered into the tissue from the coupled syringe (not shown) in the same manner as described above for the system 10. The system 110 may be provided with a stop (not shown) releasably engageable with the body 112, for example located within the enclose 144, to limit relative displacement between the first and section sections 114, 116 as a means of limiting the depth of penetration of the microneedles 118, 120.

Similarly the system 110 may be disengaged from the tissue following fluid delivery by utilising a release mechanism in the form of a pair of triggers 158, 160 to displace the first and second sections 114, 116 away from one another along an arcuate path about the pair of hinges 180 in a similar manner as described above.

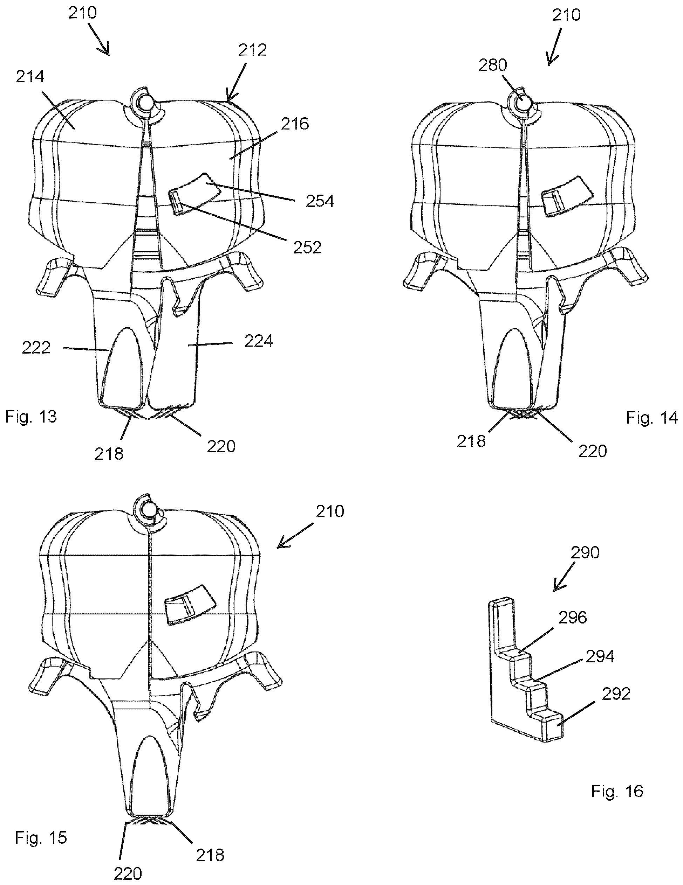

Referring to Figures 13 to 16 there is illustrated a further alternative embodiment of a microneedle based delivery system according to the present invention, generally indicated as 210. In this alternative embodiment like components have been accorded like reference numerals and unless otherwise stated perform a like function.

The delivery system 210 is effectively a variant of the system 110, comprising a body 212 having a first section 214 and a second section 216 which are again hingedly displaceable relative to one another to transition the system 210 between disengaged and engaged states. The system 210 comprises first hollow microneedles 218 provided on the first section 214 and second hollow microneedles 220 provided on the second section 216. In this embodiment the microneedles 218, 220 are of increased length relative to the previous embodiments **in** order to permit increased tissue penetration.

The body 212 defines elongate first and second arms 222, 224 at a free end of which the microneedles 218, 220 are provided. The first and second sections 214, 216 of the system 210 are secured to one another by means of a pair of hinges 280, although it will again be appreciated that numerous other configurations may be employed to enable this pivoting relative displacement. Tabs 252 provided on the first section 214 are located within windows 254 on the second section 216 to both provide a visual indication as to the level of displacement of the first and second sections 214, 216 and thus deployment of the microneedles 218, 220, and to prevent the first and second sections 214, 216 from disengaging from one another.

The system 210 is optionally provided with a stop 290 shown in isolation in Figure 16, and which in use is located within a chamber (not shown) defined between the first and second sections 214, 216 of the body 212 and arranged to selectively limit relative displacement between the first and second sections 214, 216 as a means of limiting the depth of penetration of the microneedles 218, 220. The stop 290 comprises first, second and third steps 292, 294, 296 of increasing height, whereby the stop 290 may be selectively positioned such that one of the steps 292, 294, 296 projects outwardly though the window 254 such as to be captured between the tab 252 and the window 254. The depth of the step 292, 294, 296 located in the window 254 will determine to what extent the first and second sections 214, 216 may be displaced together and thus to what extent the system 210 can be displaced into the engaged state. For example Figure 14 shows the system 210 displaced into a partially engaged state with the microneedles 218, 220 in a first relative position while Figure 15 shows the system 210 displaced into a fully engaged state with the microneedles 218, 220 in a second relative position which will achieve a greater tissue penetration than that of Figure 14. These two states may be permitted by locating different steps 292, 294, 296 of the stop 290 within the window 254. The stop 290 may also be positioned with the largest step 296 projecting through the window 254 which may be dimensioned to effectively lock the system 210 in the disengaged state to prevent accidental deployment of the microneedles 218, 220, for example during handling, particularly when applying a torque to a syringe (not shown) being coupled to the system 210.

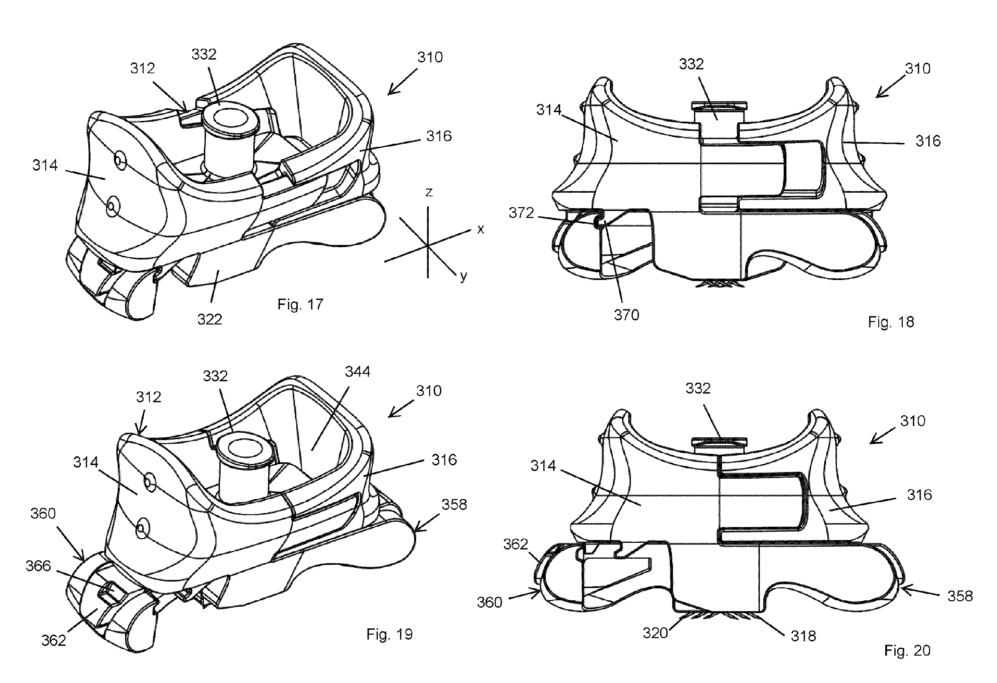

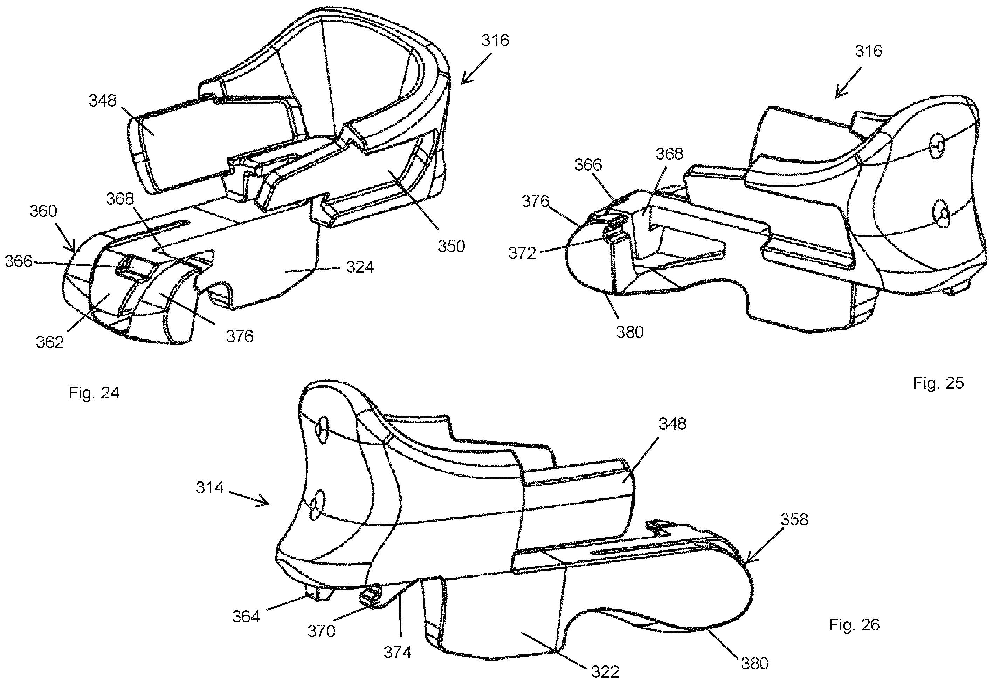

Referring to Figures 17 to 28 there is illustrated a further alternative embodiment of a microneedle based delivery system according to the present invention, generally indicated as 310. In this alternative embodiment like components have been accorded like reference numerals and unless otherwise stated perform a like function.

The delivery system 310 comprises a body 312 having a first section 314 and a second section 316 which are displaceable relative to one another to transition the system 310 between disengaged and engaged states substantially as hereinbefore described with reference to the preceding embodiments. The outer end wall of the first and second sections 314, 316 may be contoured and/or otherwise arranged to provide an ergonomic and/or retentive form factor with which a user's finger and/or thumb may be engaged during operation of the delivery system 310, in order to establish a secure hold during deployment onto the target tissue. In this way a user can securely grip the first and second sections 314, 316 between a thumb and forefinger in order to then squeeze the sections 314, 316 together to move the delivery system 310 from the disengaged to the engaged state as hereinbefore described.

The system 310 comprises first hollow microneedles 318 provided on the first section 314 and second hollow microneedles 320 provided on the second section 316. As with previous embodiments, the material, dimensions, orientation, and relative positioning of the microneedles 318, 320 may also be varied as necessary. For example the dimension and/or orientation of the microneedles 318, 320 may be arranged to provide a desired depth of insertion into the target tissue, which can therefore facilitate accurate drug delivery to specific locations or layers of tissue. Particular orientations of the microneedles 318, 320 may additionally improve the manufacturability of the delivery system 310, for example by rotation about a plane normal to the axis of the respective needle 318, 320, or a series of Euler rotations about a body-fixed coordinate system of the needle 318, 320 (where one of the axes of this coordinate system is aligned with the longitudinal axis of the needle 318, 320). Such an orientation may provide additional performance improvements whereby, during deployment additional shear strain or deformation is applied to the skin or other tissue in a plane perpendicular to the direction of motion of the first and second sections 314, 316. This can serve to further increase insertion efficiency by creating a shear strain gradient along the vertical depth of the needle 318, 320 in the skin, provide additional scratch fixation on insertion and potentially reduce pain and/or discomfort experienced by the user. Furthermore by orienting the needles 318, 320 in this way usability can be improved as deployment of the needles 318, 320 is less sensitive to slight deviation from the ideal normal orientation during insertion whereby the syringe (not shown) connected to the delivery system 310 is perpendicular to the skin.

The first and second sections 314, 316 of the body 312 are secured together but slidably displaceable relative to one another by a fixed distance to transition the system 310 between a disengaged state, for example as illustrated in Figures 17 and 18, and an engaged state, for example as illustrated in Figures 19 and 20. In the disengaged stage the microneedles 318, 320 are in a first orientation relative to one another prior to application to the target tissue, and in the engaged state are in a second orientation relative to one another. As with previously described embodiments the microneedles 318, 320 are applied against the target tissue in the disengaged state and the system 310 is then displaced into the engaged state by manually advancing the first and second section 314, 316 towards one another to draw and anchor the microneedles 318, 320 into the target tissue to allow drug delivery as hereinafter described.

The body 312 comprises an first arm 322 extending from the first section 314 and an adjacent second arm 324 extending from the second section 316 on a lower end of each of which the respective microneedles 318, 320 are provided. The shape, orientation and dimensions of the arms 322, 324 may be varied as required. The arms 322, 324 define internal delivery conduits (not shown) providing fluid communication between a delivery manifold 332 and the respective microneedles 318, 320 as hereinbefore described. The delivery manifold 332 comprises an upper inlet 334 operable for connection with a syringe (not shown) or comparable reservoir of a medium to be delivered, such as a liquid drug composition or the like, and a lower outlet 336 extending between which is a lumen 338, the lower outlet 336 and lumen 338 preferably being bifurcated (not shown) to supply both sets of microneedles 318, 320 through the respective arm 322, 324. The manifold 332 is received within an enclosure 344 defined between the first and second sections 314, 316 but which is open and thus allows a user visual access to the manifold 332 which may aid in connection or disconnection of the syringe (not shown). As described above the outlet 336, in use, is captured between the first and second sections 314, 316 in fluid tight contact with the arms 322, 324 in order to ensure a leak free delivery of fluid from the manifold 332 into the arms 322, 324 and ultimately the microneedles 318, 320. This may be achieved as hereinbefore described or by any other suitable functional alternative arrangement. The manifold comprises a baseplate 339 which articulates with tapered surfaces of enclosure 344 in such a way so as provide counter torque resistance during engagement of a leur lock syringe (not shown) to the inlet 334 and thereby isolates the outlet 336 from torque loading and thus possible misalignment with the arms 322, 324. Furthermore, the clockwise nature of the torque is strongly resisted by the interaction of the interlocked extensions 348 and guideways 350 and preventing the device 310 from being wedged apart. Another important function of the manifold 332 is to provide an abutting surface against the inner surfaces of enclosure of 344 to prevent the coming apart of the two sections 314, 316 in the "Y" direction.

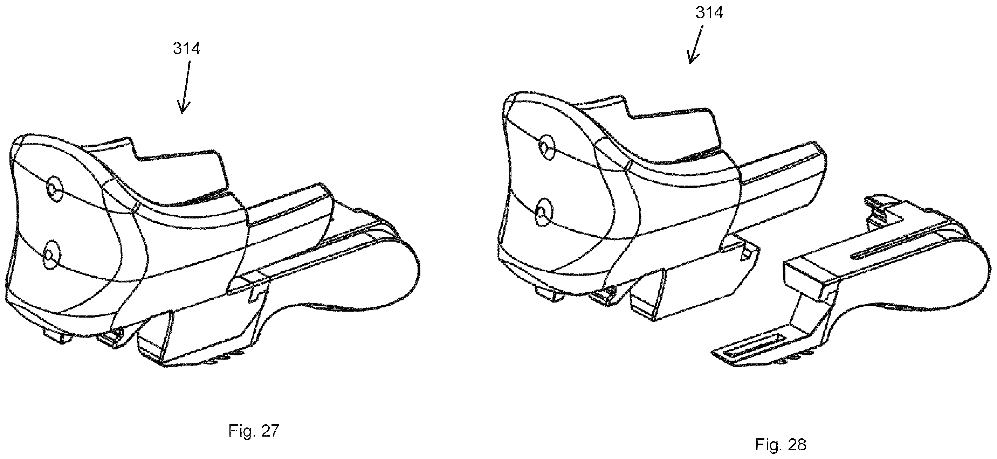

In the embodiment illustrated the first and second sections 314, 316 are the same component, but arranged so as to be inter-engagable with one another as illustrated. In particular each section 314, 316 defines a lateral extension 348 projecting from the right hand side, and a correspondingly shaped and dimensioned guideway 350 on the left hand side for receiving the extension 348 in sliding engagement therewith. In this way, with two identical parts 314, 316 facing one another the right hand extension of each section 314, 316 is receivable in the left hand guideway 350 of the opposite section 314, 316. This arrangement provides a significant improvement in manufacturing the delivery system 310 as only a single part is required to provide both sections 314, 316 but it is of course understood that the opposed sections may have different forms/geometries which still providing the above described functionality. Figures 27 and 28 illustrate an exemplary but nonlimiting form of the first section 314 as designed for manufacture, whereby the section 314 is formed from two parts which may be suitable secured together.

As with previous embodiments, the first and second arms 322, 324 are located adjacent one another, but offset in the "Y" direction on each section 314, 316, transverse to the "X" direction in which the first and second sections 314, 316 are displaceable relative to one another. In the embodiment illustrated each arm 324, 326 is offset to the right hand side so that again, when identical sections 314, 316 are facing and interlocked with one another the arms 324, 326 sit alongside one another in face to face engagement. In this way the arms 322, 324 can move alongside one another as the system 310 is transitioned into the engaged state, drawing the microneedles 318, 320 into the tissue.

In order to allow a user to withdraw the microneedles 318, 320 once a drug or the like has been dispensed into the tissue the delivery system 310 is preferably provided with a release mechanism in the form of first and second triggers 358, 360 respectively provided on the first and second sections 314, 316. Each trigger 358, 360 extends in cantilever form towards and marginally beyond the wall of the enclosure 344 of the opposite section 314, 316 in order to be accessible by the user. Each trigger 358, 360 terminates in a paddle 362 which is shaped and dimensioned for operative engagement with a finger or thumb of the user and which is independently deformable relative to the surrounding trigger 358, 360 as detailed hereinafter, in particular being formed as an independent cantilevered component captured within the cantilevered trigger 358, 360 and thus being capable of deflection independently of deflection of the trigger 358, 360.. The system 310 is preferably provided with a locking mechanism to secure the system 310 in the engaged state, in particular to avoid any inadvertent withdrawal of the microneedles 318, 320, for example while fluid is being delivered to the tissue. In the embodiment illustrated the locking mechanism is provided in the form of a protrusion or detent 364 on underside of the first and second sections 314, 316 which is in face to face engagement with the upper face of the respective trigger 358, 360 and a correspondingly shaped and dimensioned first socket 366 and second socket 368 on an upper face of the paddle 362. The first and second sockets 366, 368 are spaced apart from one another by the distance through which the first and second sections 314, 316 are displaceable between the disengaged and engaged states. The first socket 366 is located to receive the detent 364 when the system 310 is in the disengaged state, the socket 366 and detent 364 being arranged to provide a relatively low resistance to relative movement of the sections 314, 316, for example by having complimentary faces which can slide past/over one another with relative ease and which may be accommodated by deformation of the paddle 362 away from the underside of the respective section 314, 316. In this way the location of the detent 364 within the first socket 366 provides a low level of retention of the system 310 in the disengaged state which is nonetheless sufficient though to prevent inadvertent deployment of the system 310 during routine handling. When the system 310 is displaced from the disengage state to the engaged state each trigger 358, 360 and integral paddle 362 will move relative to the respective detent 364, with the paddle 362 being deflected away from the underside of the section 314, 316 to disengage the detent 364 and the first socket 366. The detent 364 will then be brought into register with the second socket 368 as the system 310 reaches the fully engaged state and will snap into engagement with the second socket 368 in order to retain the system 310 in the engaged state. The detent 364 and second socket 368 are arranged to prevent any reversal of the system 310 from the engaged back to the disengaged state without a positive action from the user as detailed hereinafter.

The system 310 may also be provided with an additional safety features to prevent inadvertent deployment into the engaged state, in particular when a syringe (not shown) is being connected. A pin (not shown) or the like could be located in a chamber or opening (not shown) provided in the space between bottom edge of the extension 348 and the upper surface of the respective trigger 358, 360 which would prevent inadvertent deployment of the system 310 until the pin is actively removed by the user. Additional or alternative safety features may of course be employed to provide this functionality.

In order to enable the first and second sections 314, 316 to be displaced away from one another to return the system 310 to the disengaged state the system 310 is gripped by the paddle 362 of each trigger 358, 360, for example between thumb and forefinger. Each paddle 362 is resiliently deformable, and the user therefore applies pressure to deform the paddles 362 in a manner which will draw the second socket 368 downwardly out of register with the detent 364. At this point the user can apply pressure to both triggers 358, 360 to effectively push the triggers 358, 360 towards one another. This will effect relative displacement between the first and second sections 314, 316 to transition the system 310 into the disengaged state, drawing the microneedles 318, 320 out of the tissue. In this configuration the detent 364 will again be aligned with the first socket 366 and once pressure is released the paddles 362 will return and the detent 364 will enter the first socket 366 to hold the system 310 in the disengaged state. The system 310 can then be withdrawn from the tissue.

The delivery system 310 may also be adapted to prevent the first and second sections 314, 316 from being pulled apart or away from one another beyond the disengaged state, in order to ensure that a user does not inadvertently effect such a displacement. The system 310 may therefore comprise a retention lock comprising a second projection or detent 370 provided on the underside of the respective section 314, 316 and a corresponding third socket 372 provided in the respective trigger 358, 360. The second detent 370 and third socket 372 are positioned to be engaged when the system 310 is in the disengaged state, for example as seen in Figure 18, and move away from one another as the sections 314, 316 are displaced into the engaged state, thus providing no resistance to this movement. However, the shape and configuration of the detent 370 and socket 372 is such that they engage when the sections 314, 316 are in the disengaged state and prevent the sections 314, 316 from being further separated from one another.

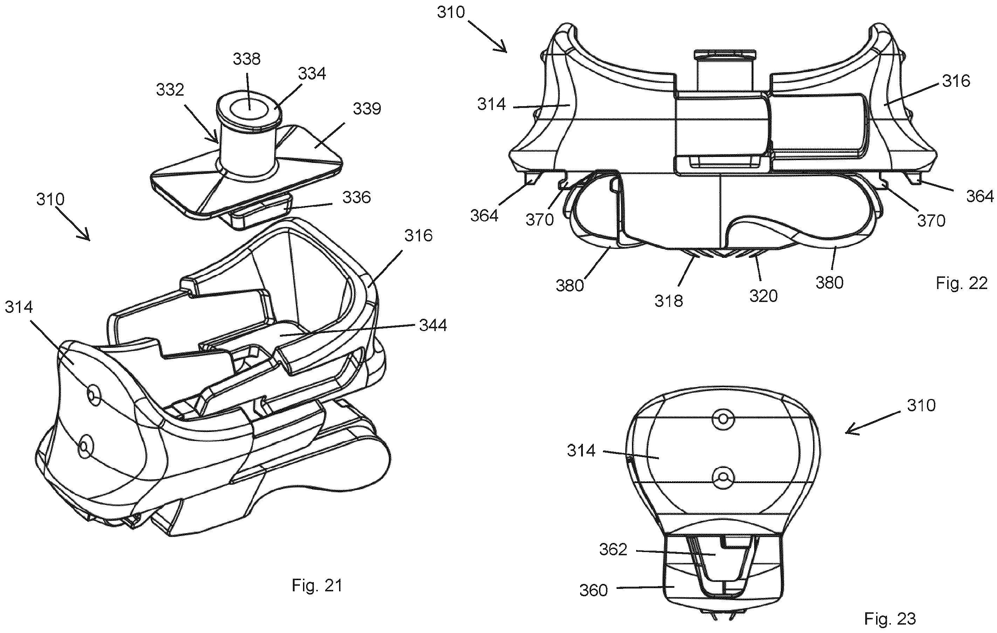

However in order to allow the two identical halves or sections 314, 316 to be initially brought into register with one another, for example from the initial position of engagement shown in Figure 22, each trigger 358, 360 must be able to pass over the second detent 370 as the sections 314, 316 are brought together towards the initial disengaged state from being completely separated from one another. In the embodiment illustrated this is facilitated by providing complementary surfaces, the second detent 370 with a sloping or ramped surface 374 while the respective contacting portion of the trigger 358, 360 comprises a curved or sloping surface 376. These surfaces contact as the sections 314, 316 are initially brought together, and as the trigger 358, 360 is of cantilevered form it will be forced to deflect downwardly away from the second detent 370 as the sections 314, 316 are advanced towards the disengaged state, thus effectively defining a single stage ratchet arrangement. In the particularly preferred arrangement illustrated the portion of the trigger 358, 360 defining the sloped surface 376 is cantilevered in both a longitudinal or "X" direction in addition to a transverse or "Y" direction and is therefore readily deflected to allow the trigger 358, 360 to pass the second detent 370. As the first and second sections 314, 316 reach the disengaged position or state the sloped surface 376 of the trigger 358, 360 will have passed the second detent 370 and will then return to the pre-deflected position, causing the second detent 370 to be captured in the third socket 372 and thereby preventing the two sections 314, 316 from being reversely separated beyond the disengaged state. At this point the detent 364 will also be located in the first socket 366 thereby lightly retaining the delivery system 310 in the disengaged state ready for use.

As detailed hereinbefore, and in greater detail in International patent applications WO2018/069543 and WO2019/201903, the relative displacement of the microneedles 318; 320 when engaged with tissue effects a particular deformation of the target tissue, applying a shear force in order to improve penetration and anchoring of the microneedles 318, 320. In order to further improve this action, the delivery device 310 is provided with a pair of tissue contacting feet 380 which are conveniently formed on or defined by an underside or tissue contacting face of the triggers 358, 360. The feet may however be provided as separate components. The feet 380 are preferably arranged such as to have a lower surface that is approximately aligned with the microneedles 318, 320, namely to be at approximately the same depth or "Z" dimension. In this manner, when the delivery device 310 is initially applied to the skin in the disengaged state, the pair of feet 80 will also contact the skin, preferably at locations longitudinally spaced from one another in the "X" direction and beyond the microneedles 318, 320. Then as the sections 314, 316 are displaced towards the engaged state the pair of feet 380 will be displaced longitudinally away from one another and from the microneedles 318, 320 in the "X" direction, which will act to apply tension to the intervening skin and thereby improving the penetrating efficacy of the microneedles 318, 320. The skin contact made by the pair of feet 380 protects the skin surrounding and beneath the microneedles 318, 320 from over compression by dissipating any excess load applied to the delivery system 310 by the user, reducing the injection pressure requirements and improving injectability. The tissue contacting surface of the feet 380 may be conditioned or otherwise adapted to increase friction with the skin or other tissue in order to further increase the functionality thereof.

The delivery system 310 may also be provided with an additional safety system to prevent inadvertent deployment into the engaged state, in particular when the system 310 is being handled and introduced onto the skin surface. The feet 380 and triggers 358, 360 may comprise a cantilevered element (not shown) that in response to the downwards pressure and reactive forces applied by the skin to the feet 380 moves an incorporated detent (not shown) out of register with a recess on the underside of the respect section 314, 316 thereby allowing the system 310 to be transitioned from the disengaged to engaged states in a load-responsive manner.

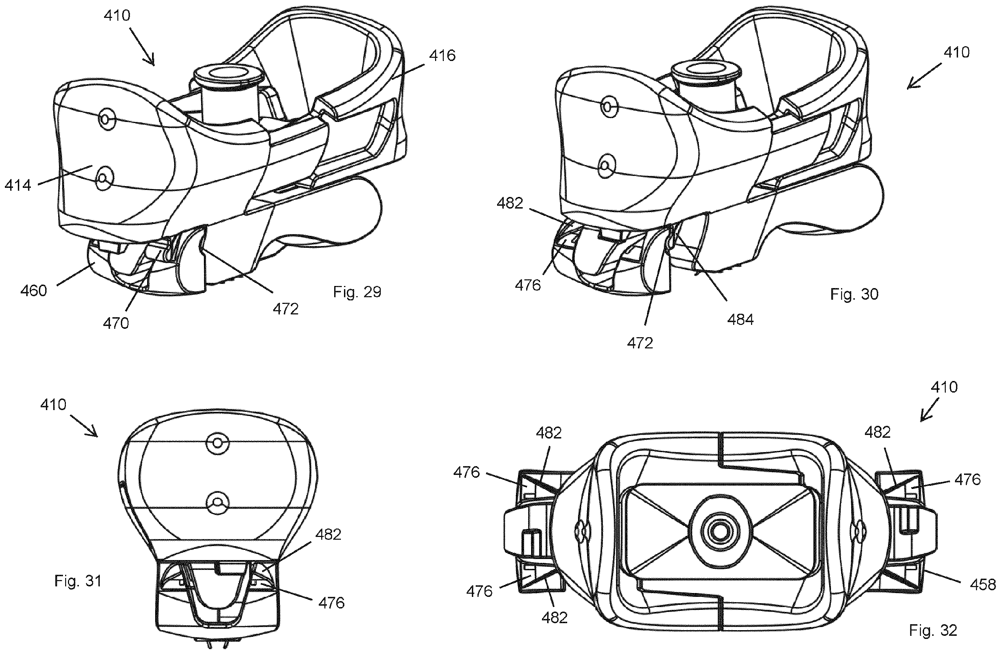

Figures 29 to 32 illustrate a further embodiment of a microneedle based delivery system according to the present invention and generally indicated as 410. In this embodiment like components have been accorded like reference numerals and unless otherwise stated perform a like function. The delivery system 410 mirrors the configuration and general operation of the system 310 shown in Figures 17 to 28, with one modification to improve the operability thereof. Specifically, in this embodiment, the delivery system 410 comprises a second detent 470 and first and second triggers 458, 460 which are modified to define angled/tapered surfaces 482 adjacent and perpendicular to a sloped surface 476 and which flare inwardly in the "Y" direction. During the initial engagement of first and second sections 414, 416 the second detent 470 contacts both the sloped surface 476 and the tapered surface 482 in order to force the trigger 458, 460 to deflect outwardly in the both the "Y" direction and partially downwardly in the "Z" direction during displacement of the first and second sections 414, 416 from the initial separated state towards the disengaged state as hereinbefore described. The second detent includes an outer flared surface 484 which meshes with the tapered surface 482 to allow the second detent 470 and trigger 458, 460 to slide past one another in contact and effecting the lateral deformation of the trigger 458, 460. In this way, the extent of cantilever bending of the trigger 458, 460 in the sagittal plane to produce the required clearance for the second detent 470 is significantly reduced. The flexural rigidity of the triggers 458, 460 in the removal plane can therefore be increased, improving manufacturability but also reducing the extent of trigger deflection during removal, and ensuring that the second detent 470 engages the third socket 472 to prevent the system 410 from being pulled apart. Furthermore, as shown most clearly in Figure 32, the cut-out on the trigger 458, 460 defined by the sloped and tapered surfaces 476, 482 is mirrored about a central plane conveniently creating a pair of triangle- or arrow-shaped features. These will by virtue of the configuration and operation of the device 410 be hidden from the user in the detached configuration, but come into view only when the device 410 is deployed into the engaged state. Coloured indicia such as arrow heads (not shown) or the like may be provided on the sloped surfaces 476 to further highlight this aspect. With or without colour coding, this can improve usability by intuitively indicating the manner of removal to the user, in addition to improving the grip, feel and handling of the device 410.

### Examples of Experimental Results

Prototypes of the above described embodiments of the hollow microneedle delivery system 10; 110; 210; 310; 410 according to the invention were produced to assess the initial manufacturability, function and injectability of the delivery system in a series of in vitro and in vivo experiments, the results of which are set out hereinafter.

### Example 1 - Production of Prototype of an Embodiment of the Invention

Fully functional, high-fidelity prototypes of an above embodiment of the current invention were manufactured using commercially-available stainless steel 31G hypodermic needles (Microfine^{™}, Becton Dickinson & Company, USA) embedded into individual rapid-prototyped parts produced using a resin-based 3D printing system (Photon Mono, AnyCubic, China; z-axis resolution of 25µm and x-y spot size of 48µm). The main body and manifold were produced in an ABS-like Photopolymer Grey Resin (Elegoo, China), whilst a flexible resin (eResin-Flex, eSUN, China) was used to produce deformable seals integrated into the underside of the manifold for achieving a fluid-tight junction. The prototyped devices exhibited an array of six 31G microneedles in a 2 x 3 configuration, each forming a 27° angle with the substrate, with an 820µm vertical tip height from the substrate and 1,500µm interspacing. Spacing between the lateral rows was 2,000µm and relative linear travel between them of no more than 4,600µm permitted, corresponding to displacement to transition the system from the detached (disengaged) to the attached (engaged) state in skin. Initial inspection and testing was used to confirm that all hypodermic needles were in fluid communication with the manifold and that a fluid-tight seal was achieved prior to release for testing as described below.

### Example 2 - In vitro Injectability Assessment of a Low Viscosity Preparation in Porcine Skin

The following study was performed to assess the injectability of prototypes of the invention prepared according to the above compared to a control Mantoux technique (using a 27G hypodermic needle) and comparator device (NanoSoft^{tm}, NanoPass, Israel) for delivering low and high volumes of a low viscosity material into ex vivo skin samples. (The NanoSoft^{™} is an injection device with three 0.6mm, hollow, pyramidal-shaped silicon crystal microneedles (approximately 80µm lumen diamater) which is applied to the skin at a 45°angle). Freshly harvested full-thickness porcine skin samples (approximately 20cm x 20cm) were placed over a 1.5cm thick silicone suturing model and secured to an underlying cork board using pins. A low viscosity (approximately 1 centipoise (cp)) solution for injection was prepared by dissolving Methylene Blue (1%) in Phosphate Buffered Saline (PBS) Solution and adding flurescent beads (in a 1-;10 dilution). N=3 injections per material volume (0.1ml and 0.5ml) per injection method (Mantoux, NanoSoft^{™} device and present invention) were administered to the skin and evaluated. An additional 1ml group was performed for the control Mantoux technique and present invention only, due to challenges encountered with delivering this volume of material with the NanoSoft^{™} device in pilot testing. Devices were used to perform a single injection only and not re-used. Injectability (including ease of deployment and removal, leakage and skin bleb formation) was qualitatively assessed, whilst tissue disruption and injection distribution were qualitatively assessed using Optical Coherence Tomography (OCT), cryosectioning and histological analysis.

### Results

The injection process was straightforward for 0.1ml of a 1cp solution for all devices. However, with increasing injection volume a commensurate increase in injection backpressure was encountered for the NanoSoft^{™} and Mantoux injections, but not the devices according to the present invention. This, combined with the anchorage during injection achieved by the present invention were noted as differentiating attributes which contributed to an overall improved injection experience for the user over the control and comparator devices. Macroscopic images show the distribution in the epidermis/upper dermis for NanoSoft^{™} devices and more dermal distribution for Mantoux and injections using the present invention. There was no significant damage to the skin surface at the injection sites for any of the devices. Skin sectioning and histological analysis confirmed confined distribution of injected material in the upper dermis/epidermis for the NanoSoft^{™}, and greater dermal distribution for injections using the present invention and Mantoux injections, which is most likely attributable to the combined effects of a relatively smaller vertical microneedle height (i.e. 600µm) and steeper application angle (i.e. 45° to the skin surface) of the NanoSoft^{™} device resulting in a relatively shallow injection deposition . Microdisruptions were noticed in the upper dermis when high volumes (i.e. 0.5ml) were injected with NanoSoft^{™}. Tissue damage at the injection site and in the lower dermis were seen for Mantoux injections. Minimal microdisruptions were observed at the injection sites for the present invention and in the dermis, even when the high volumes were injected (500ul and 1ml) although there was some tissue damage, as expected. The injected solution was observed to distribute further away from the injection site for the current invention compared to both the Mantoux and NanoSoft^{™} injections.

### Example 3 - In vitro Injectability Assessment of a High Viscosity Preparation in Porcine Skin

A further set of experiments were conducted using the devices and materials from Example 2 to deliver 3 volumes (0.1ml, 0.5ml and 1ml) of a high viscosity preparation (average zero shear viscosity of 3 samples using the Cross Model determined to be 3830±610cp (mean±SD)) to porcine skin, in vitro. This solution (Formulation B) was prepared in the following way; 20mls of Formulation A (prepared by mixing 45ml Viscosity Standard (18.8 cPs at 20°C) (VWR Chemicals), 5ml Methylene Blue Solution and 1ml Brij 30 surfactant) mixed with 2ml of 30nm yellow-green fluorescent beads. N=1 injections per injection volume (0.1ml, 0.5ml and 1ml) per device (Mantoux Technique, NanoSoft^{™}and the current invention) were administed to porcine skin, in vitro. Fresh devices were used to perform each single injection only and not re-used. Injectability (including ease of deployment and removal, leakage and skin bleb formation) was qualitatively assessed.

### Results

The present invention was associated with superior injectability (most noticeably in the form of overall injection success, significantly reduced back pressure as well as speed of injection) at all three injection volumes. Whilst the Mantoux technique (with a 27G hypodermic needle) was able to successfully deliver at all three volumes, it was associated with relatively increased back pressure and all injections had to be performed slowly. The NanoSoft^{™} device was routinely associated with the highest backpressure and slowest injection time at all injection levels, and was not able to be used to administer more than 0.5ml of the high viscosity solution. For all injection techniques and devices, a commensurate increase in injection backpressure with increasing injection volumes was observed. Additionally, the resulting bleb formations in the skin did not appear to disperse as far as the low viscosity formulations from the low viscosity experiments (Example 2), and it is postulated that this likely had an influence on back pressure generation. As in Example 2, superior injectability coupled with anchorage during injection achieved by the current invention were noted as differentiating attributes which contributed to an overall improved injection experience for the user over the control and comparator devices.

### Example 4 - In vivo Injection of a Sterile Solution into a Healthy Volunteer

A prototype of an embodiment of the current invention prepared according to the procedure above was sterilised in a 70% alcohol solution in a first step. A sterile syringe containing 3mls of 0.9% Sodium Chloride for injection BP (BBraun, Germany) was attached to the syringe coupling. Using their non-dominant hand the volunteer self-applied the device using a clicking action to a site on the dominant (right) forearm, which had been cleaned and sterilised in advance using an alcohol-based preparation. With the device anchored to the skin, the non-dominant hand was then transferred to and used to grip the syringe flange and plunger and administer the injection. The volunteer experience and macroscopic images of the site taken at regular intervals for up to 2 hours following injection were qualitatively assessed as measures of injectability of an embodiment of the current invention.

### Results

The volunteer was able to manipulate the device and attach the device to their right forearm, administer the full 3mls of sterile solution and remove the device using their non-dominant hand without any issues. The volunteer reported only mild pain on application of the device (1 on a visual analogue scale (VAS) of 0 - 10) and likewise reported a very mild and transient 'heat' sensation during the very initial injection phase (1 on the VAS). Resistance to injection was observed to drop off dramatically following this initial, immediate phase of injection and the injection could be performed with minimal effort and sensation (0 -1 on the VAS), the entire volume of 3mls taking less than 20s to administer in this initial demonstration (although the volunteer reported that it was felt that this could have been performed more rapidly). The injection was associated with a bleb formation that was observed to radiate in all directions away from the microneedle attachment site, resulting in a bleb approximately 25-30mm in diameter and approximately 2-4mm in height (directly above the microneedle insertion site). No leakage at the manifold of the device or at the injection site was noted. Macroscopic images showed significant reduction in size of the bleb between 35 and 80 minutes, with the bleb being virtually indistinguishable from the surrounding skin by 2 hours following the injection, save for the slight erythema associated with the micro-disruptions created by the inserted microneedles. No adverse reactions to the application of the microneedles or administration of the sterile solution were noted, and the erythema associated with the microneedle insertion was observed to resolve within 1-2 days.

The microneedle based delivery system 10, 110; 210; 310; 410 of the present invention thus provides a means of effecting the highly targeted delivery of a fluid such as a drug to a target site, allowing accurate surface placement and depth selection, while being simple to operate and permitting connection to a conventional syringe, and having particular utility in ocular treatment by facilitating ease of engagement and anchoring while also allowing the accurate delivery of a drug or vaccine to a desired depth in the skin or layer of the eye such as the subchoroidal or suprachoroidal regions.

The invention is not limited to the embodiments described herein but can be amended or modified without departing from the scope of the present invention.

## Claims

1. A microneedle based delivery system (10; 110; 210; 310; 410) comprising a body (12; 112; 212; 312) having a first section (14; 114; 214; 314; 414) and a second section (16; 116; 216; 316; 416) displaceable relative to one another; at least one first hollow microneedle (18; 118; 218; 318) provided on the first section and at least one second hollow microneedle (20; 120; 220; 320) provided on the second section, the first and second sections being displaceable relative to one another in order to transition the microneedles between a disengaged state and an engaged state; a delivery manifold (32; 132; 332) in fluid communication with the first and second hollow microneedles; wherein the first and second section of the body each define an elongate arm (22; 122; 222; 322; 422; 24; 124; 224) at a free end of which the respective at least one microneedle is provided, **characterized in that** each arm defines an upper end opposite the lower end, and a fluid flow path extending through the arm between the upper and lower end, the delivery manifold being in fluid communication with the fluid flow path at the upper end of each arm.

2. The microneedle based delivery system of claim 1 in which a longitudinal axis of the at least one first microneedle extends at a first oblique angle relative to the direction of displacement between the first and second sections, and a longitudinal axis of the at least one second microneedle extends at a second oblique angle relative to the direction of displacement between the first and second sections.

3. The microneedle based delivery system of claim 2 in which the first oblique angle extends away from the second oblique angle.

4. The microneedle based delivery system of claim 2 or 3 in which the at least one first microneedle is transversely offset to the at least one second microneedle relative to the direction of displacement between the first and second sections.

5. The microneedle based delivery system of any preceding claim in which the first and the second sections are slidably and/or hingedly displaceable relative to one another.

6. The microneedle based delivery system of any preceding claim in which the delivery manifold is captured between the first and second sections at least when the microneedles are in the engaged state.

7. The microneedle based delivery system of claim 6 in which the delivery manifold is clamped against the body when the microneedles are in the engaged state such as to establish a fluid tight seal between the delivery manifold and the body.

8. The microneedle based delivery system of any preceding claim in which the delivery manifold comprises an inlet (34; 334) adapted for connection with a fluid reservoir and an outlet engagable with the body such that the inlet is in fluid communication with the hollow microneedles.

9. The microneedle based delivery system of claim 8 in which the body defines a chamber (40) between the first and second sections in which the outlet of the delivery manifold is captured and which chamber is arranged to bias the outlet into sealing engagement with the body at least when the system is in the engaged state.

10. The microneedle based delivery system of any preceding claim in which the body defines an enclosure (44) at least partially surrounding the delivery manifold.

11. The microneedle based delivery system of any preceding claim in which the delivery manifold is in fluid communication with the end of the fluid flow paths when the microneedles are in both the engaged and disengaged state.

12. The microneedle based delivery system of any preceding claim comprising a release mechanism (58; 158; 358; 60; 160; 360) defined by a trigger on each of the first and second sections and arranged to facilitate manual displacement of the system into the disengaged state.

13. The microneedle based delivery system of any preceding claim comprising a stop (290) releasably engageable with the body to limit relative displacement between the first and section sections.

14. The microneedle based delivery system of any preceding claim comprising a pair of tissue contacting feet (380) each defining a tissue contacting surface which is substantially longitudinally aligned with the microneedles.

15. The microneedle based delivery system of claim 14 comprising interlocking elements operable to prevent the first and second sections from being displaced from the disengaged to the engaged state when the elements are interlocked, and which are separable in response to downward pressure and reactive forces applied to the feet by contacted tissue.

## Patentansprüche

1. Mikronadelbasiertes Verabreichungssystem (10; 110; 210; 310; 410), umfassend einen Körper (12; 112; 212; 312), der einen ersten Abschnitt (14; 114; 214; 314; 414) und einen zweiten Abschnitt (16; 116; 216; 316; 416) aufweist, die relativ zueinander verschiebbar sind; mindestens eine erste hohle Mikronadel (18; 118; 218; 318), die an dem ersten Abschnitt bereitgestellt ist, und mindestens eine zweite hohle Mikronadel (20; 120; 220; 320), die an dem zweiten Abschnitt bereitgestellt ist, wobei der erste und zweite Abschnitt relativ zueinander verschiebbar sind, um die Mikronadeln zwischen einem gelösten Zustand und einem in Eingriff stehenden Zustand übergehen zu lassen; einen Verabreichungsverteiler (32; 132; 332) in Fluidverbindung mit der ersten und zweiten hohlen Mikronadel; wobei der erste und zweite Abschnitt des Körpers jeweils einen länglichen Arm (22; 122; 222; 322; 422; 24; 124; 224) definieren, an dessen freiem Ende die jeweilige mindestens eine Mikronadel bereitgestellt ist, **dadurch gekennzeichnet, dass** jeder Arm ein dem unteren Ende gegenüberliegendes oberes Ende und einen sich durch den Arm zwischen dem oberen und unteren Ende erstreckenden Fluidströmungsweg definiert, wobei der Verabreichungsverteiler am oberen Ende jedes Arms in Fluidverbindung mit dem Fluidströmungsweg steht.

2. Mikronadelbasiertes Verabreichungssystem nach Anspruch 1, bei dem sich eine Längsachse der mindestens einen ersten Mikronadel in einem ersten schiefen Winkel relativ zu der Richtung der Verschiebung zwischen dem ersten und zweiten Abschnitt erstreckt, und sich eine Längsachse der mindestens einen zweiten Mikronadel in einem zweiten schiefen Winkel relativ zu der Richtung der Verschiebung zwischen dem ersten und zweiten Abschnitt erstreckt.

3. Mikronadelbasiertes Verabreichungssystem nach Anspruch 2, bei dem sich der erste schiefe Winkel von dem zweiten schiefen Winkel weg erstreckt.

4. Mikronadelbasiertes Verabreichungssystem nach Anspruch 2 oder 3, bei dem die mindestens eine erste Mikronadel zu der mindestens einen zweiten Mikronadel relativ zu der Richtung der Verschiebung zwischen dem ersten und zweiten Abschnitt in Querrichtung versetzt ist.

5. Mikronadelbasiertes Verabreichungssystem nach einem der vorhergehenden Ansprüche, bei dem der erste und der zweite Abschnitt gleitend und/oder gelenkig relativ zueinander verschiebbar sind.

6. Mikronadelbasiertes Verabreichungssystem nach einem der vorhergehenden Ansprüche, bei dem der Verabreichungsverteiler zwischen dem ersten und zweiten Abschnitt gefangen ist, mindestens wenn sich die Mikronadeln in dem in Eingriff stehenden Zustand befinden.

7. Mikronadelbasiertes Verabreichungssystem nach Anspruch 6, bei dem der Verabreichungsverteiler gegen den Körper geklemmt ist, wenn sich die Mikronadeln in dem in Eingriff stehenden Zustand befinden, um so eine fluiddichte Abdichtung zwischen dem Verabreichungsverteiler und dem Körper herzustellen.

8. Mikronadelbasiertes Verabreichungssystem nach einem der vorhergehenden Ansprüche, bei dem der Verabreichungsverteiler einen Einlass (34; 334), der für Verbindung mit einem Fluidreservoir angepasst ist, und einen Auslass umfasst, der so mit dem Körper in Eingriff bringbar ist, dass der Einlass in Fluidverbindung mit den hohlen Mikronadeln steht.

9. Mikronadelbasiertes Verabreichungssystem nach Anspruch 8, bei dem der Körper eine Kammer (40) zwischen dem ersten und zweiten Abschnitt definiert, in der der Auslass des Verabreichungsverteilers gefangen ist, und welche Kammer dafür angeordnet ist, den Auslass in abdichtenden Eingriff mit dem Körper vorzuspannen, mindestens wenn sich das System in dem in Eingriff stehenden Zustand befindet.

10. Mikronadelbasiertes Verabreichungssystem nach einem der vorhergehenden Ansprüche, bei dem der Körper eine Umschließung (44) definiert, die den Verabreichungsverteiler mindestens teilweise umgibt.

11. Mikronadelbasiertes Verabreichungssystem nach einem der vorhergehenden Ansprüche, bei dem der Verabreichungsverteiler mit dem Ende der Fluidströmungswege in Fluidverbindung steht, wenn sich die Mikronadeln sowohl im in Eingriff stehenden als auch im gelösten Zustand befinden.

12. Mikronadelbasiertes Verabreichungssystem nach einem der vorhergehenden Ansprüche, umfassend einen Freigabemechanismus (58; 158; 358; 60; 160; 360), der durch einen Auslöser an jedem des ersten und zweiten Abschnitts definiert und dafür angeordnet ist, manuelle Verschiebung des Systems in den gelösten Zustand zu erleichtern.

13. Mikronadelbasiertes Verabreichungssystem nach einem der vorhergehenden Ansprüche, umfassend einen Anschlag (290), der lösbar mit dem Körper in Eingriff bringbar ist, um relative Verschiebung zwischen dem ersten und section-Abschnitt zu begrenzen.

14. Mikronadelbasiertes Verabreichungssystem nach einem der vorhergehenden Ansprüche, umfassend ein Paar von gewebekontaktierenden Füßen (380), die jeweils eine gewebekontaktierende Oberfläche definieren, die im Wesentlichen in Längsrichtung mit den Mikronadeln ausgerichtet ist.

15. Mikronadelbasiertes Verabreichungssystem nach Anspruch 14, umfassend ineinandergreifende Elemente, die betriebsfähig sind, zu verhindern, dass der erste und zweite Abschnitt vom gelösten in den in Eingriff stehenden Zustand verschoben werden, wenn die Elemente ineinandergreifen, und die in Reaktion auf Abwärtsdruck und Reaktionskräfte trennbar sind, die durch kontaktiertes Gewebe auf die Füße ausgeübt werden.

## Revendications

1. Système d'administration basé sur des micro-aiguilles (10 ; 110 ; 210 ; 310 ; 410) comprenant un corps (12 ; 112 ; 212 ; 312) ayant une première section (14 ; 114 ; 214 ; 314 ; 414) et une seconde section (16 ; 116 ; 216 ; 316 ; 416) déplaçables l'une par rapport à l'autre ; au moins une première micro-aiguille creuse (18 ; 118 ; 218 ; 318) prévue sur la première section et au moins une seconde micro-aiguille creuse (20 ; 120 ; 220 ; 320) prévue sur la seconde section, les première et seconde sections étant déplaçables l'une par rapport à l'autre afin de faire passer les micro-aiguilles d'une position désengagée à une position engagée ; un collecteur d'administration (32 ; 132 ; 332) en communication fluidique avec les première et seconde micro-aiguilles creuses ; dans lequel la première et la seconde section du corps définissent chacune un bras allongé (22 ; 122 ; 222 ; 322 ; 422 ; 24 ; 124 ; 224) à l'extrémité libre duquel est prévue l'au moins une micro-aiguille respective, **caractérisé en ce que** chaque bras définit une extrémité supérieure opposée à l'extrémité inférieure, et un chemin d'écoulement de fluide s'étendant à travers le bras entre l'extrémité supérieure et l'extrémité inférieure, le collecteur d'administration étant en communication fluidique avec le chemin d'écoulement de fluide à l'extrémité supérieure de chaque bras.

2. Système d'administration basé sur des micro-aiguilles selon la revendication 1, dans lequel un axe longitudinal de l'au moins une première micro-aiguille s'étend selon un premier angle oblique par rapport à la direction du déplacement entre les première et seconde sections, et un axe longitudinal de l'au moins une seconde micro-aiguille s'étend selon un second angle oblique par rapport à la direction du déplacement entre les première et seconde sections.

3. Système d'administration basé sur des micro-aiguilles selon la revendication 2, dans lequel le premier angle oblique s'étend à partir du second angle oblique.

4. Système d'administration basé sur des micro-aiguilles selon la revendication 2 ou 3, dans lequel l'au moins une première micro-aiguille est décalée transversalement par rapport à l'au moins une seconde micro-aiguille par rapport à la direction du déplacement entre les première et seconde sections.

5. Système d'administration basé sur des micro-aiguilles selon une quelconque revendication précédente, dans lequel les première et seconde sections sont déplaçables par glissement et/ou par charnière l'une par rapport à l'autre.

6. Système d'administration basé sur des micro-aiguilles selon une quelconque revendication précédente, dans lequel le collecteur d'administration est capturé entre les première et seconde sections au moins lorsque les micro-aiguilles sont en position engagée.

7. Système d'administration basé sur des micro-aiguilles selon la revendication 6, dans lequel le collecteur d'administration est serré contre le corps lorsque les micro-aiguilles sont en position engagée, de manière à établir un joint étanche entre le collecteur d'administration et le corps.

8. Système d'administration basé sur des micro-aiguilles selon une quelconque revendication précédente, dans lequel le collecteur d'administration comprend une entrée (34 ; 334) adaptée pour la connexion à un réservoir de fluide et une sortie pouvant être engagée avec le corps de sorte que l'entrée soit en communication fluidique avec les micro-aiguilles creuses.

9. Système d'administration basé sur des micro-aiguilles selon la revendication 8, dans lequel le corps définit une chambre (40) entre les première et seconde sections dans laquelle la sortie du collecteur d'administration est capturée et laquelle chambre est agencée pour forcer la sortie à s'engager avec le corps au moins lorsque le système est en position engagée.

10. Système d'administration basé sur des micro-aiguilles selon une quelconque revendication précédente, dans lequel le corps définit une enceinte (44) entourant au moins partiellement le collecteur d'administration.

11. Système d'administration basé sur des micro-aiguilles selon une quelconque revendication précédente, dans lequel le collecteur d'administration est en communication fluidique avec l'extrémité des chemins d'écoulement de fluide lorsque les micro-aiguilles sont à la fois en position engagée et désengagée.

12. Système d'administration basé sur des micro-aiguilles selon une quelconque revendication précédente, comprenant un mécanisme de libération (58 ; 158 ; 358 ; 60 ; 160 ; 360) défini par un déclencheur sur chacune des première et seconde sections et agencé pour faciliter le déplacement manuel du système en position désengagée.

13. Système d'administration basé sur des micro-aiguilles selon une quelconque revendication précédente, comprenant une butée (290) pouvant être engagée de manière amovible avec le corps pour limiter le déplacement relatif entre les première et seconde sections.

14. Système d'administration basé sur des micro-aiguilles selon une quelconque revendication précédente, comprenant une paire de pieds de contact tissulaire (380) définissant chacun une surface de contact tissulaire qui est sensiblement alignée longitudinalement avec les micro-aiguilles.

15. Système d'administration basé sur des micro-aiguilles de la revendication 14, comprenant des éléments imbriqués fonctionnant pour empêcher les première et seconde sections d'être déplacées de la position désengagée à la position engagée lorsque les éléments sont imbriqués, et qui sont séparables en réponse à la pression vers le bas et aux forces réactives appliquées aux pieds par les tissus en contact.
